# EUROPÄISCHE PATENTSCHRIFT

(11) **EP 2 339 994 B1**
(45) Veröffentlichungstag und Bekanntmachung des Hinweises auf die Patenterteilung: **20.04.2016**
(21) Anmeldenummer: 09780989.1
(22) Anmeldetag: 23.07.2009
(51) Int. Cl.: A61F 2/30, A61F 2/34, A61F 2/46

(54) **POSITIONIERHILFE IN FORM EINER SELBSTFIXIERENDEN PROBEHÜFTGELENKPFANNE**
POSITIONING AID IN THE FORM OF A SELF-AFFIXING TEST HIP JOINT SOCKET
ACCESSOIRE DE POSITIONNEMENT SOUS FORME D'IMPLANT D'ACÉTABULUM À AUTO-FIXATION

(30) Priorität: 01.08.2008 DE 102008036036; 17.09.2008 DE 102008047627
(43) Veröffentlichungstag der Anmeldung: 06.07.2011
(73) Patentinhaber: Smith & Nephew Orthopaedics AG, 6340 Baar (CH)
(72) Erfinder: ZUMSTEG, Lukas, CH-5085 Sulz (CH); KILCHENMANN, Thomas, CH-8810 Horgen (CH)
(74) Vertreter: Popp, Eugen
(86) Internationale Anmeldenummer: PCT/EP2009/059502
(87) Internationale Veröffentlichungsnummer: WO 2010/012648

(56) Entgegenhaltungen:
- EP-A- 0 051 359
- EP-A- 0 612 509
- EP-A- 1 852 091
- WO-A-98/34557
- GB-A- 2 159 416

## Beschreibung

Die Erfindung betrifft eine Positionierhilfe in Form einer selbstfixierenden Probehüftgelenkpfanne.

Hüftgelenkpfannen sind ein Bestandteil herkömmlicher Hüftgelenkprothesen, die einen Oberschenkelschaft, der in den Oberschenkelknochen implantiert wird und einem Kugel-gelenkkopf, der auf dem Oberschenkelschaft verankert ist, umfassen. Der Kugelgelenkkopf ist dabei drehbar in der Hüftgelenkpfanne gelagert. Die Hüftgelenkpfanne wird in der Regel in das Hüftbecken implantiert, wobei ein schalenförmiger, metallischer Verankerungskörper implantiert wird, in welchem ein Kunststoffkörper, in welchem die Hüftkugel artikuliert, eingesetzt wird.

Die Hüftgelenkpfanne - wie beispielsweise in der DE 197 14 050 A1 beschrieben - wird üblicherweise mittels eines oder mehrerer Verankerungszapfen oder einer oder mehrerer Schrauben fest mit dem Hüftknochen verbunden. Weitere Formen der Verankerung der Pfanne im Knochen sind die Fixierung mittels eines Schraubengewindes auf der Schale (EP 0601 224B1) oder die Fixierung mittels eine Presssitzes, erzielt durch ein gezieltes Übermaß des metallischen Schalenkörpers gegenüber dem vorbereiteten knöchernen Lager.

Die relative Position von Hüftgelenkpfanne und Hüftgelenkschaft bestimmt den Bewegungsumfang des Gelenkes, in welchem dieses ohne Kollision zwischen Schenkelhals und Pfannenrand bewegt werden kann. Während der Hüftgelenkschaft in seiner Position im Wesentlichen durch die morphologischen Bedingungen des Oberschenkelknochens festgelegt ist, ist die Orientierung der Pfanne im Becken frei wählbar. Um die Gefahr einer Dislokation des Hüftgelenkkopfes oder die Beschädigung der Pfanne aufgrund einer Kollision (= Impingement) zwischen einem Schenkelhals und Pfannenrand zu vermeiden bzw. zumindest zu verringern, werden während der Implantation der einzelnen Komponenten der Hüftprothese Kollisions-und Dislokationstests durchgeführt. Der Gefahr eines Impingements kann auch durch Verändern der Länge des Schenkelhalses, entgegengewirkt werden. Diesbezüglich wird beispielhaft auf die DE 10 2006 045 358 A1 hingewiesen, in der ein verstellbarer Schenkelhals beschrieben wird. Nach erfolgter Implantation der metallischen Pfannenschale lässt sich die Impingement- und Dislokationsgefahr nur noch bedingt durch die Verwendung von Inlays mit unterschiedlicher Randgestaltung beeinflussen. Zur Auswahl und Positionsbestimmung des definitiven Inlays werden wieder verwendbare Probeinlays verwendet, welche in der implantierten Pfannenschale leicht lösbar positioniert werden.

Durch das Verwenden eines einstellbaren Schenkelhalses oder die Verwendung verschiedener Inlays kann eine mangelhafte Positionierung oder Orientierung der Hüftgelenkpfanne entsprechend nur teilweise korrigiert werden. Diese Korrektur ist jedoch relativ aufwändig und wird insgesamt als unzureichend empfunden. Wird die Korrektur durch die Verwendung verschiedener Inlays mitbestimmt, so wird es zudem als nachteilhaft angesehen, dass die Wahl des Inlays unter Gesichtspunkten erfolgt, die deren Variationsmöglichkeit einschränkt.

Die WO 98/34557 A1 betrifft eine Vorrichtung für die Positionierung eines Implantats. In einer Ausführungsform sind Mittel vorgesehen, um eine temporäre Verbindung des Implantats mit dem Knochen zu ermöglichen, so dass das Implantat als Probeimplantat genutzt werden kann. Die EP 1 852 091 A1 offenbart ein Set aus einer Gelenkpfanne und einer Probepfanne, wobei die Ausdehnung eines Flansches der Probepfanne ähnlich zu der Hüftgelenkpfanne ist und einen Vorsprung an ihrer äußeren Oberfläche umfasst.

Der vorliegenden Erfindung liegt die Aufgabe zu Grunde, eine Positionierhilfe zur Implantation eines Pfannenimplantates einer Hüftgelenkprothese aufzuzeigen, mit der die Gefahr einer Dislokation wirkungsvoll verringert wird, wobei die Belastung für den Patienten während der Operation möglichst gering sein soll. Diese Aufgabe wird durch eine Positionierhilfe in Form einer selbstfixierenden Probehüftgelenkpfanne (im Folgenden Probepfanne genannt), welche mit einem Manipulierinstrument bedient werden kann, nach Patentanspruch 1 gelöst. Des Weiteren wird die Aufgabe durch eine Anordnung, umfassend die selbstfixierende Probepfanne und ein Manipulierinstrument nach Anspruch 9, eine Anordnung, umfassend die selbstfixierende Probepfanne und ein

Navigationssystem mit einer Positionsbestimmungshilfe und/oder Orientierungsbestimmungshilfe nach Anspruch 10 und eine Anordnung, umfassend eine selbstfixierende Probepfanne und ein Inlay, insbesondere Probeinlay, sowie ein Set, bestehend aus einer Vielzahl von Probepfannen nach Anspruch 12 gelöst.

Die Erfindung beruht auf dem Gedanken eine selbstfixierende Probepfanne zum lösbaren Einsetzen in eine entsprechende Ausnehmung eines Hüftknochens anzugeben. Die selbstfixierende Probepfanne umfasst dabei eine Außenfläche, die an die knöcherne Ausnehmung angepasst ist, eine Innenfläche, die an ein Inlay, insbesondere Probeinlay und/oder einen Kugelgelenkkopf zur Aufnahme desselben in der selbstfixierenden Probepfanne angepasst ist und mindestens ein Halteelement zum Fixieren der selbstfixierenden Probepfanne in der Ausnehmung, wobei das mindestens eine Halteelement mindestens ein Federelement umfasst, das derart ausgebildet ist, dass das Federelement im eingesetzten Zustand eine nach außen gerichtete Federkraft zumindest abschnittsweise auf die Ausnehmung des Hüftknochens zum lösbaren Fixieren der selbstfixierenden Probepfanne ausübt.

Die selbstfixierende Probepfanne kann mit einem Probeinlay, welches mit dem Kugelkopf des Hüftschaftes artikuliert, ausgestattet sein. Da Kugelköpfe mit unterschiedlichen Durchmessern in Anwendung sind, kann das Probeinlay auswechselbar mit einer selbstfixierenden Probepfanne, z.B mit einer Schraubengewinde im Pol der selbstfixierenden Probepfanne verbunden werden. Üblicherweise werden Kugelköpfe mit Durchmessern von 22, 28, 32 oder 36 mm angeboten. Entsprechend können Probeinlays in diesen Durchmessern, welche in verschiedene selbstfixierende Probepfannen eingesetzt werden können, bereitgestellt werden.

Nach Auswahl und Montage des entsprechenden Probeinlays kann die selbstfixierende Probepfanne mittels eines Manipulierinstrumentes in der vorbereiteten Aussparung des Knochens positioniert und durch Relaxieren des/der Federelements/Federelemente, beispielsweise einer oder mehrerer Blattfedern fixiert werden.

Das Federelement übt eine Haltekraft auf die selbstfixierende Probepfanne aus, so dass diese innerhalb der Ausnehmung des Hüftknochens fixiert ist. Die selbstfixierende

Probepfanne kann durch Aufbringen einer der Federkraft bzw. Haltekraft entgegen wirkenden Kraft mit dem Manipulierinstrument leicht in der Ausnehmung des Hüftknochens positioniert und aus dieser wieder entfernt werden. Dadurch kann die selbstfixierende Probepfanne wiederholt besonders exakt positioniert und orientiert werden. So können nach dem Einsetzen der selbstfixierenden Probepfanne in die Ausnehmung des Hüftknochens mit dem Manipulierinstrument verschiedene Tests durchgeführt werden, beispielsweise Impingement- und Dislokationstests oder einen Test des Bewegungsspielraums des Oberschenkelknochens. Aufgrund der Ergebnisse dieser Tests kann dann, wenn diese positiv verlaufen, die definitive Position und Orientierung der selbstfixierenden Probepfanne festgelegt oder, wenn die Resultate der Tests nicht zufriedenstellend sind, Position und/oder Orientierung entsprechend geändert werden. Diese Prozedur kann beliebig oft wiederholt werden, bis ein zufriedenstellendes Resultat erreicht ist, wobei aufgrund des Einsatzes des Federelementes eine Schädigung des Hüftknochens vermieden wird. Insbesondere werden den Hüftknochen schädigende Fixierelemente wie Stifte oder Schrauben nicht benötigt. Nach Ermittelung der definitiven Position und Orientierung mit der selbstfixierenden Probepfanne wird dann das Pfannenimplantat in der so gefundenen Position und Orientierung implantiert.

Das Übertragen der so gefundenen Position und Orientierung auf das Implantat kann beispielsweise mit Hilfe eines chirurgischen Navigationssystems sicher erfolgen. Dazu wird beispielsweise ein entsprechender Positionsgeber auf dem Manipulierinstrument platziert, welches mit der selbstfixierenden Probepfanne verbunden sein kann. Auf einem Manipulierinstrument des Pfannenimplantates kann dann ebenfalls ein Positionsgeber platziert werden. So kann die Position des Pfannenimplantates exakt analog zu der als optimal gefundenen Position der selbstfixierenden Probepfanne gewählt werden. Alternativ kann die Position an Hand von Markierungen, welche an der knöchernen Aussparung angebracht werden, reproduziert werden. Diese Markierungen bezeichnen z.B. den Verlauf des Randes der selbstfixierenden Probepfanne in der gefundenen optimalen Position. Der Rand des definitiven Pfannenimplantates kann dann so gewählt werden, dass dieser mit der Markierung in Deckung gebracht wird.

Die vorliegende selbstfixierenden Probepfanne hilft also wirkungsvoll und den Patienten wenig belastend das Risiko einer Dislokation oder eines Impingements zu vermeiden.

In einer bevorzugten Ausführungsform ist das Federelement bzw. mindestens eines der Federelemente als Blattfeder ausgebildet, die vorzugsweise zumindest bereichsweise die Außenfläche der selbstfixierenden Probepfanne bildet. Ein derartiges Federelement ist in der Herstellung besonders einfach und nimmt innerhalb der selbstfixierenden Probepfanne nur einen geringeren Platz ein.

Vorzugsweise ist ein proximales Ende der Blattfeder/der Blattfedern in der Nähe des Durchstoßpunktes einer Symmetrieachse der selbstfixierenden Probepfanne an dieser fixiert und ein in einem Randbereich der selbstfixierenden Probepfanne befindliches, distales Ende durch Druckbeaufschlagung in seiner Position veränderbar. Bei einer derartigen Ausgestaltung der Blattfeder wird diese während des Einsetzvorgangs gespannt. Dadurch können zwei Vorgänge, nämlich das Einsetzen der selbstfixierenden Probepfanne und das Spannen der Blattfeder gleichzeitig stattfinden.

Vorzugsweise weist das Halteelement bzw. weisen die Halteelemente an einem dem Hüftknochen zugewandten Flächenabschnitt der Außenfläche der selbstfixierenden Probepfanne Erhebungen, insbesondere zackenförmige Erhebungen auf. Dies verbessert die Verankerung der selbstfixierenden Probepfanne in der Ausnehmung des Hüftknochens.

Die Erhebungen sind vorzugsweise an dem distalen Ende der Blattfeder angeordnet. Im Bereich des distalen Endes wirkt ein relativ hoher Druck, weshalb die Erhebungen in diesem Bereich besonders effektiv sind.

In einer konkreten Weiterbildung der selbstfixierenden Probepfanne ragt ein Überstand im eingesetzten Zustand vorzugsweise mindestens 0,5 cm, insbesondere 1,0 cm über eine Wand der Ausnehmung des Hüftknochens hinaus. Ein derartiger Überstand bietet eine Angriffsfläche, um die selbstfixierende Probepfanne mittels eines Manipulierinstruments ohne großen Aufwand aus der Ausnehmung des Hüftknochens zu nehmen.

Vorzugsweise sind die Halteelemente im Bereich des Überstandes derartig ausgestaltet, dass sie mit einem entsprechenden Manipulierinstruments zum Lösen der Halteelemente in Eingriff bringbar sind. Dies erleichtert das Herausnehmen der selbstfixierenden Probepfanne.

In einer bevorzugten Ausführungsform weist die selbstfixierende Probepfanne durchgehende Aussparungen derartig auf, dass eine gitterähnliche Struktur ausgebildet ist. Dadurch kann der Chirurg visuell beurteilen, ob die selbstfixierende Probepfanne an der vorbereiteten Aussparung im Knochen eng anliegt und ausreichend knöcherne Unterstützung für die später zu implantierende Metallschale vorhanden ist.

Die oben genannte Aufgabe wird des Weiteren auch durch eine Anordnung, umfassend die selbstfixierenden Probepfanne und ein Manipulierinstrument gelöst, wobei das Manipulierinstrument derart, insbesondere zangenartig ausgebildet ist, dass durch eine das Manipulierinstrument bedienende Person oder Vorrichtung eine Kraft auf die Halteelemente aufbringbar ist, welche in das Innere der selbstfixierenden Probepfanne gerichtet ist. Durch den Einsatz des Manipulierinstruments, insbesondere für den Fall, dass das Manipulierinstrument zangenartig ausgebildet ist, kann eine relativ hohe Kraft auf die Halteelemente aufgebracht werden, was die Verwendung von relativ "harten" Blattfedern ermöglicht.

Mit anderen Worten wird die oben genannte Aufgabe weiterhin durch eine Anordnung, umfassend die selbstfixierende Probepfanne und ein Navigationssystem mit einer Positionsbestimmungshilfe und/oder Orientierungsbestimmungshilfe gelöst, wobei die Positionsbestimmungshilfe und/oder Orientierungsbestimmungshilfe zur Bestimmung und Abspeicherung der Position und/oder Orientierung der selbstfixierenden Probepfanne an diese fixierbar ist/sind. Durch eine derartige Anordnung kann die Position und/oder Orientierung der selbstfixierenden Probepfanne genau bestimmt werden. Dies ist vorteilhaft, wenn die selbstfixierende Probepfanne lediglich eine Testfunktion einnimmt, d.h. nach der Bestimmung der geeigneten Position und Orientierung durch ein Pfannenimplantat ersetzt wird, das für einen dauerhaften Einsatz besser geeignet ist. Dieses letztgenannte Pfannenimplantat kann dann auch über Schrauben, Verankerungszapfen, oder dergleichen dauerhaft mit dem Hüftknochen verbunden werden.

Weiterhin wird die Aufgabe auch durch eine Anordnung, umfassend die selbstfixierende Probepfanne und ein Inlay, insbesondere Probeinlay, welches in die selbstfixierende Probepfanne einsetzbar oder eingesetzt ist, gelöst. Durch eine derartige Anordnung können auch verschiedene Probeinlays getestet werden.

Schließlich wird die Aufgabe auch durch ein Set, bestehend aus einer Vielzahl von selbstfixierenden Probepfannen gelöst, wobei sich die einzelnen selbstfixierenden Probepfannen in ihrer Größe, insbesondere bezüglich ihres Kugelradius unterscheiden. Das Bereitstellen eines derartigen Sets ermöglicht es, nicht nur die Position und Orientierung der selbstfixierenden Probepfanne und damit letztlich des Pfannenimplantates exakt einzustellen, sondern auch deren geometrische Ausgestaltung verschiedenen Tests zu unterwerfen. Durch Bereitstellen verschiedener Inlays, insbesondere Pobeinlays kann dieses Set noch erweitert werden.

Weitere Ausführungsformen ergeben sich aus den Unteransprüchen.

Nachfolgend wird die Erfindung hinsichtlich weiterer Merkmale und Vorteile anhand von Ausführungsbeispielen beschrieben, die anhand der beigefügten schematischen Zeichnungen näher erläutert werden.

Hierbei zeigen:
- Fig. 1: eine Schrägansicht einer selbstfixierenden Probepfanne nach einer ersten Ausführungsform;
- Fig. 2: die selbstfixierende Probepfanne nach der ersten Ausführungsform mit eingesetztem Probeinlay in Schrägansicht;
- Fig. 3: die Ausführungsform nach Fig. 1 und 2 in geschnittener Ansicht;
- Fig. 4: Schnitt entlang der Linie III-III aus Fig. 3 mit einer ersten Stellung eines Halteelements;
- Fig. 5: Schnitt entlang der Linie III-III aus Fig. 3 mit einer zweiten Stellung des Halteelements;
- Fig. 6: Schrägansicht eines Probeinlays;
- Fig. 7: Schnittansicht des Probeinlays aus Fig. 6;
- Fig. 8a - Fig. 11a: Schrägansichten verschiedener Ausführungsformen des Probeinlays;
- Fig. 8b - Fig. 11b: Schnittansichten der Probeinlays aus Fig. 8a - 10a;
- Fig. 12: eine Probehüftgelenkpfanne und ein Manipulierinstrument (teilweise in geschnittener Ansicht); und
- Fig. 13: ein Flußdiagramm.

In der nachfolgenden Beschreibung werden für gleiche und gleich wirkende Teile dieselben Bezugsziffern verwendet.

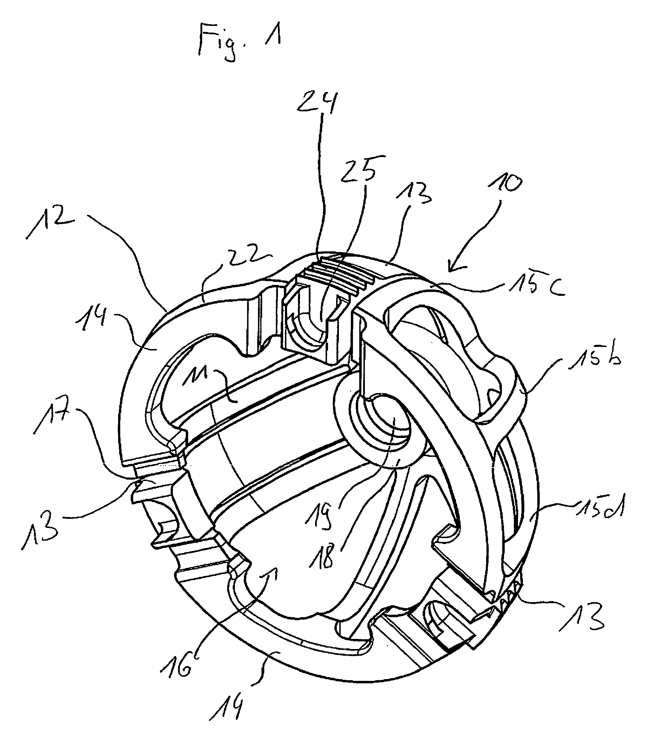

Fig. 1 zeigt eine selbstfixierende Probepfanne in Schrägansicht. Die Form der selbstfixierenden Probepfanne entspricht in etwa einer halben Hohlkugel. Eine Außenfläche 10 liegt im eingesetzten Zustand der selbstfixierenden Probepfanne zumindest bereichsweise an eine Ausnehmung eines Hüftknochens (nicht in den Figuren gezeigt) an. Eine Innenfläche 11 dient der Aufnahme eines Inlays oder eines Kugelgelenkkopfes (Inlay und Kugelgelenkkopf nicht in den Figuren gezeigt).

Die selbstfixierende Probepfanne umfasst einen Grundkörper 12 und drei Halteelementen 13. Es können alternativ auch vier oder fünf bzw. eine beliebige Anzahl von Halteelementen (nicht in Figuren gezeigt) vorgesehen sein.

Der Grundkörper 12, welcher vorzugsweise aus einem Metall oder einer einer metallischen Legierung, insbesondere aus Stahl, vorzugsweise Instrumentenstahl, oder einer Titanlegierung mit ausreichenden Federeigenschaften gefertigt ist, bildet einen etwa kreisförmigen Polabschnitt 18 und drei weitere Abschnitte 14. Der Polabschnitt 18 weist eine kreisförmige Aussparung 19 auf, die beispielsweise der Fixierung eines Inlays (nicht in den Figuren gezeigt) dienen kann. Die drei Abschnitte 14 bestehen aus vier Stegen 15a bis d. Die Stege 15a bis 15c verlaufen in etwa in meridionaler Richtung und werden durch den Steg 15d, welcher sich äquatorial im Bereich einer Pfannenöffnung 16 erstreckt, miteinander verbunden. Die Abschnitte 14 des Grundkörpers 12 sind voneinander beabstandet, wobei sich in Zwischenräumen 17, die seitlich durch die Abschnitte 14 begrenzt werden, die Halteelemente 13 befinden.

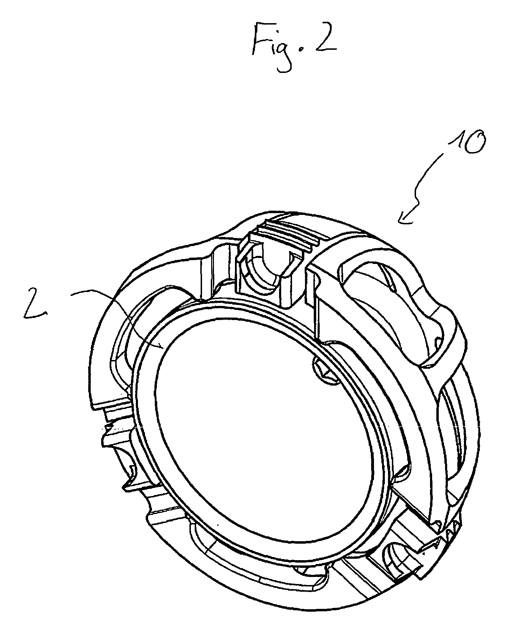

Die Halteelemente 13 sind nochmals gesondert als schematischer Schnitt entlang der Radialrichtung in Figur 2 gezeigt. Ein proximales Ende 20 des Halteelements 13 ist mit dem Polabschnitt 18 (siehe Figur 1) des Grundkörpers 12 fest verbunden. Ein distales Ende 21 befindet sich frei stehend in einem Randbereich 22 der selbstfixierenden Probepfanne.

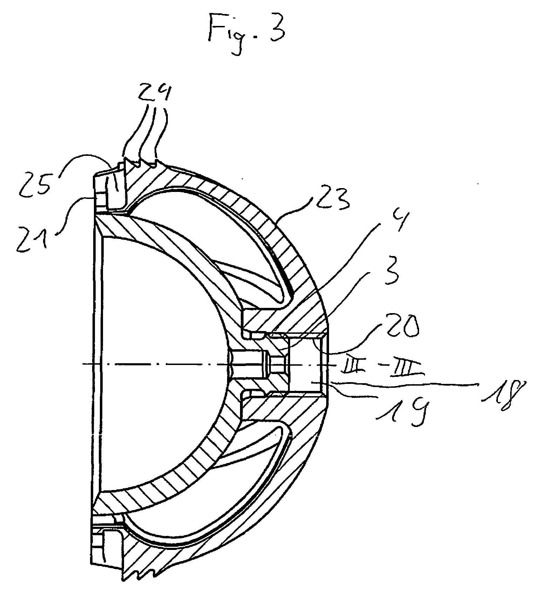

Das Halteelement 13 (siehe auch Figur 3) umfasst eine Blattfeder 23. Weitere Bestandteile des Halteelements 13 sind die zackenförmigen Erhebungen 24 sowie die Aussparung 25. Die zackenförmigen Erhebungen 24 sind am distalen Ende 21 des Halteelements 13 angebracht und weisen in Richtung der Ausnehmung des Hüftknochens. Die Aussparung 25 ist am distalen Ende 21 des Halteelements 13 im Bereich eines Überstandes (im eingesetzten Zustand) der selbstfixierenden Probepfanne angeordnet.

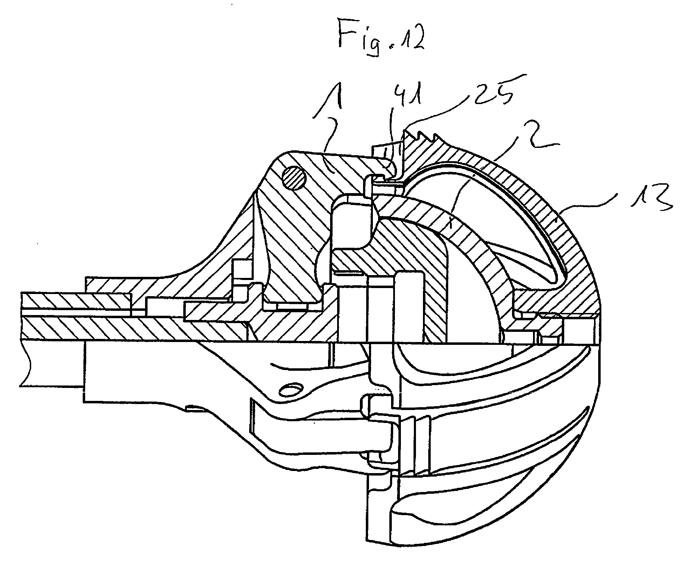

Die zackenförmigen Erhebungen 24 erhöhen die Haltekraft zwischen selbstfixierender Probepfanne und der Ausnehmung des Hüftknochens, in welche die selbstfixierende Probepfanne einzusetzen ist. Die Aussparung 25 dient beispielsweise als Gegenstück zu einem entsprechenden zangenartigen Manipulierinstrument 1 (S. Fig. 12), mit dessen Hilfe eine Kraft (radial) nach innen auf die drei Halteelemente 13 ausgeübt werden kann. Auch können Positions- und/oder Orientierungshilfen als Bestandteile eines Navigationssystems an den jeweiligen Aussparungen 25 der Halteelemente 13 befestigt werden. Die Positions- und Orientierungshilfen können aber auch andersartig, beispielsweise an den Stegen 15d der Abschnitte 14 montiert sein.

Die selbstfixierende Probepfanne ist lediglich zu Testzwecken während der Operation ausgebildet. Dauerhaft im Patienten wird dann ein Pfannenimplantat eingesetzt. Dieses Pfannenimplantat kann unlösbar mit dem Hüftknochen, beispielsweise durch Zementieren, verbunden werden.

In den Figuren 2 bis 5 ist die Hüftgelenkpfanne aus Fig. 1 mit eingesetztem Probeinlay 2 zu erkennen. Mit dem Probeinlay kann dann ein Gelenktest mit Impingement-Kontrolle durchgeführt werden. Die Probepfanne wird also vorzugsweise in Kombination mit dem Probeinlay verwendet.

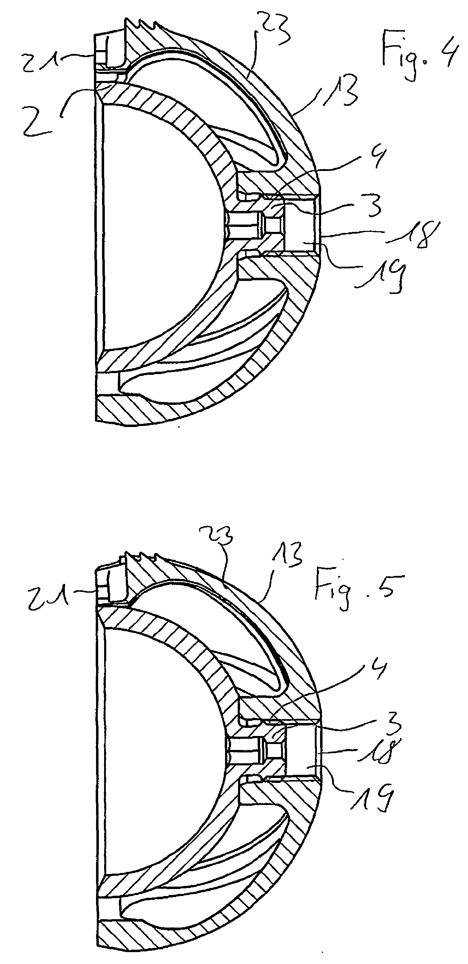

In den Figuren 4 und 5 sind zwei verschiedene Positionen des Halteelements 13 mit der Blattfeder 23 zu sehen. In Fig. 4 ist das distale Ende 21 des Halteelements 13 von dem Probeinlay 2 beabstandet. In einer solchen Position befindet sich die Anordnung, bestehend aus Probepfanne und Probeinlay 2, beispielsweise, wenn die Probepfanne in der Ausnehmung des Hüftknochens eingesetzt ist. In Fig. 5 wiederum ist das distale Ende 21 des Halteelements 13 gegenüber Fig. 4 radial nach innen gedrückt. Befinden sich alle drei Halteelemente 13 (von denen nur eines in Fig. 4 und 5 zu sehen ist) in dieser Position, kann die Probepfanne einschließlich Probeinlay aus der Ausnehmung des Hüftknochens entfernt werden.

Das Probeinlay 2 (siehe Figuren 3 bis 5) ist bevorzugt über ein Befestigungselement 3 in der Aussparung 19 des Polabschnitts 18 der Probepfanne befestigt. Im vorliegenden Fall weist das Befestigungselement 3 ein Außengewinde 4 auf, über welches das Probeinlay 2 mit der Probepfanne verschraubt werden kann. Das Außengewinde 4 kann beispielsweise aus Stahl hergestellt sein.

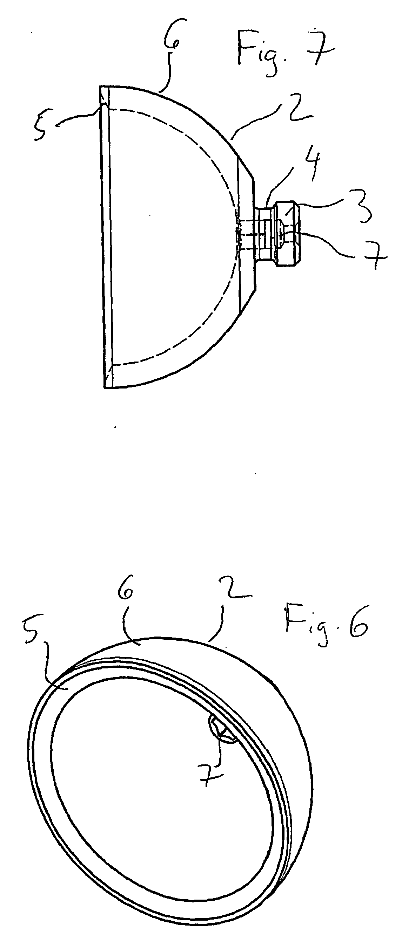

In den Figuren 6 und 7 ist das Probeinlay 2 nochmals isoliert dargestellt. Neben dem Befestigungselement 3 mit dem Außengewinde 4 und einem Hohlraum 7 ist die Ausgestaltung eines Grundkörpers 6 mit einem Rand 5 zu sehen. Der Grundkörper 6 ist ebenso wie der Grundkörper 12 der Probepfanne (nicht in Figuren 6 und 7 zu sehen) halbhohlkugelförmig ausgeformt und weist keine Ausnehmungen auf. Der Rand 5 ist radial nach innen abgeschrägt.

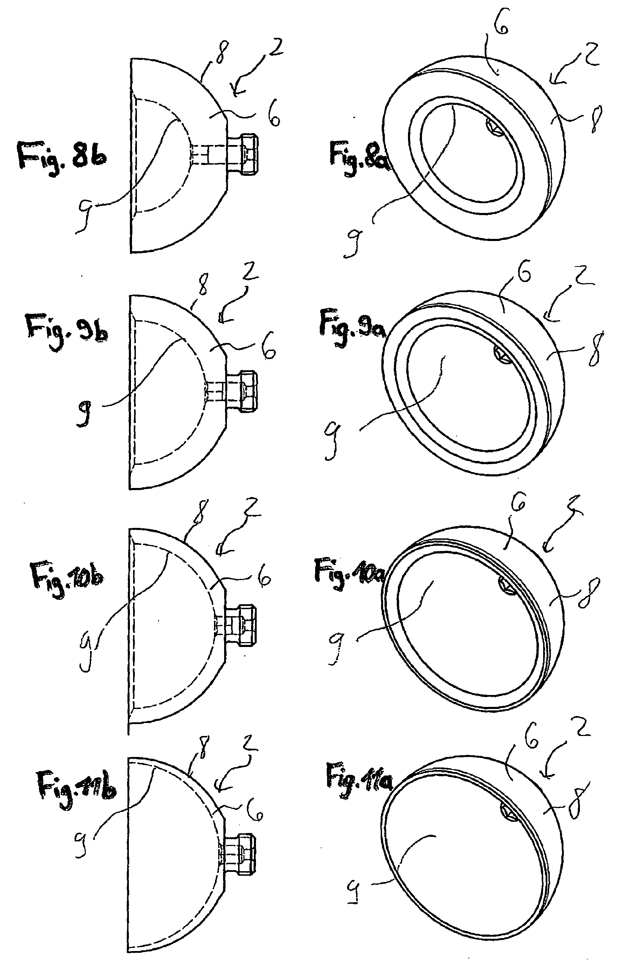

In den Figuren 8a bis 11a sowie den Figuren 8b bis 11b sind vier verschiedene Ausführungsformen des Probeinlays 2 dargestellt. Eine Außenfläche 8 des Grundkörpers 6 ist bei allen vier Ausführungsformen gleich, so daß die vier gezeigten Probeinlays in die gleiche Probepfanne eingesetzt werden können. Prinzipiell ist es natürlich auch vorstellbar, Probeinlays mit verschiedenen Außenflächen 8 zu realisieren, um verschiedene Probepfannen den erwähnten Tests unterziehen zu können.

Vorzugsweise werden Inlays mit Kalottendurchmessern von 22 mm, 28 mm, 32 mm oder 36 mm hergestellt. Das Inlay mit einem Kalottendurchmesser von 22 mm ergibt den kleinsten Bewegungsumfang und das Inlay mit einem Kalottendurchmesser von 36 mm den größten Bewegungsumfang. Besonders vorteilhaft ist es, wenn gleichzeitig, beispielsweise während der Operation, ein ganzes Set aus verschiedenen Probeinlays zur Verfügung steht. Während der Operation wird in die Probeinlays vorzugsweise eine speziell angefertigte Manipulierhüftkugel (nicht in den Figuren gezeigt) eingesetzt.

In Fig. 12 ist die Probepfanne mit eingesetztem Probeinlay gezeigt, wobei das Manipulierinstrument 1 mit der Probepfanne in Eingriff ist. Genauer gesagt, sind Greifelemente 41 in die Aussparungen 25 der Halteelemente 13 eingepaßt. Das Manipulierinstrument 1 ist also so ausgebildet, daß die Probepfanne mit eingesetztem Probeinlay positioniert und entfernt werden kann. Vorteilhaft ist also neben einer stabilen Position, welche über die Verschraubung im Polabschnitt 18 (siehe Figuren 3 bis 5) erreicht werden kann, auch eine einfache Auswechselbarkeit durch das Manipulierinstrument 1. Durch das Manipulierinstrument 1 ist eine Kraft radial nach innen auf die Probepfanne aufbringbar.

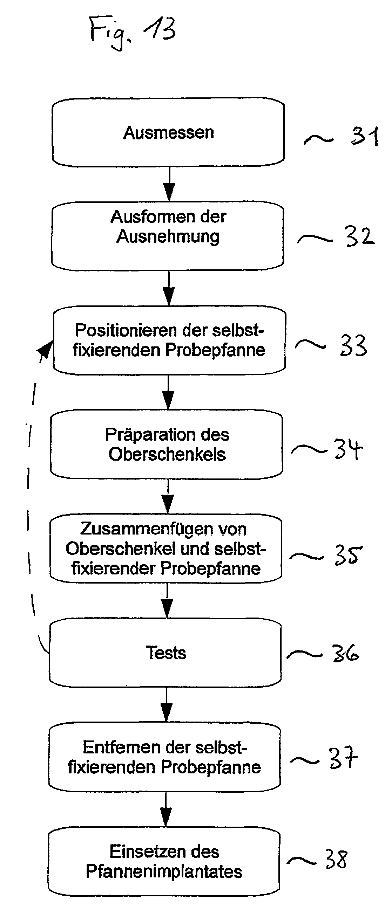

Figur 13 zeigt ein Flußdiagramm, das den typischen Verlauf einer Hüftoperation unter Zuhilfenahme der in Fig. 1 und 2 beschriebenen selbstfixierenden Probepfanne illustriert. Die hier beschriebene Operation läßt sich in 8 Teilschritte (dargestellt durch die Blocks 31 bis 38) unterteilen.

### Schritt 1 (Block 31):

Im ersten Schritt wird der Hüftbereich des Patienten ausgemessen, wobei Position und Orientierung der einzelnen Bestandteile bezüglich des Operationstisches gespeichert werden. Im Folgenden bezieht sich der Begriff Orientierung/Position stets auf den Operationstisch als Referenz.

### Schritt 2 (Block 32):

In diesem (optionalen) Schritt wird der Hüftknochen des Patienten derart vorbearbeitet, dass die selbstfixierende Probepfanne einsetzbar ist.

### Schritt 3 (Block 33):

Die selbstfixierende Probepfanne wird nun unter Berücksichtigung der Messwerte aus Schritt 1 in die Ausnehmung des Hüftknochens eingesetzt, wobei über eine Positionsund Orientierungsbestimmungseinrichtung bzw. ein Navigationssystem die Position und Orientierung der selbstfixierenden Probepfanne bestimmt und abgespeichert wird. Stimmt die Position nicht mit der in Schritt 1 errechneten Position überein, so kann die selbstfixierende Probepfanne mit Hilfe eines entsprechenden Manipulierinstruments gelöst und repositioniert werden.

### Schritt 4 (Block 34):

Es wird ein Oberschenkelschaft in den Oberschenkel getrieben und anschließend über den Schenkelhals mit dem Kugelgelenkkopf verbunden.

### Schritt 5 (Block 35):

Im Folgenden wird der Kugelgelenkkopf in die selbstfixierende Probepfanne eingesetzt. Zuvor kann gegebenenfalls aus einer Reihe von Inlays ein passendes Inlay ausgewählt und in die selbstfixierende Probepfanne eingesetzt werden.

### Schritt 6 (Block 36):

Nach dem Zusammenfügen von Oberschenkel und selbstfixierender Probepfanne werden verschiedene Tests, wie beispielsweise ein Bewegungsspielraum-Test, Impingement-Test und ein Luxations-Test durchgeführt. Verlaufen diese Tests mit zufriedenstellendem Ergebnis, so schließt sich nach Schritt 6 der siebte Schritt an. Verlaufen die Ergebnisse nicht zufriedenstellend, so geht der Operateur zu Schritt 3 zurück und verändert die Position der selbstfixierenden Probepfanne.

### Schritt 7 (Block 37):

Mit einem geeigneten Manipulierinstrument, welches auch zum Positionieren der selbstfixierenden Probepfanne dienen kann, wird diese nun entfernt. Selbstverständlich ist es hier besonders wichtig, die exakte Position und Orientierung der selbstfixierenden Probepfanne zu bestimmen.

### Schritt 8 (Block 38):

In diesem Schritt wird unter Einhaltung der zuvor bestimmten Idealposition der selbstfixierenden Probepfanne ein Pfannenimplantat implantiert. Dieses Pfannenimplantat wird beispielsweise einzementiert und/oder mit dem Hüftknochen verschraubt.

In Schritt 3 (Positionieren der selbstfixierenden Probepfanne) ist es auch denkbar, verschiedene selbstfixierende Probepfannen, die sich beispielsweise in ihrem Radius unterscheiden, einzusetzen und in Schritt 6 zu testen. Genauso ist es im Schritt 5 denkbar verschiedene Inlays, insbesondere Probeinlays bereitzustellen, um diese in Schritt 6 zu testen.

Alternativ ist es auch möglich, in die Ausnehmung des Hüftknochens eine Zwischenschale einzusetzen, in welche die selbstfixierende Probepfanne und/oder das Pfannenimplantat positioniert wird/werden.

Besonders vorteilhaft ist es, wenn die selbstfixierende Probepfanne einen gewissen Überstand bezüglich der Ausnehmung des Hüftknochens aufweist. Dadurch wird es erleichtert, das Manipulierinstrument und/oder eine Positionsbestimmungshilfe und/oder eine Orientierungsbestimmungshilfe in Eingriff mit der selbstfixierenden Probepfanne zu bringen.

Auch wenn die vorliegende Erfindung vorzugsweise zum Einsatz im menschlichen Körper vorgesehen ist, kann diese ebenfalls bei Tieren, insbesondere Hunden zum Einsatz kommen und entsprechend ausgebildet sein.

### Bezugszeichenliste

- 1: Manipulierinstrument
- 2: Probeinlay
- 3: Befestigungselement
- 4: Außengewinde
- 5: Rand (des Probeinlays)
- 6: Grundkörper (des Probeinlays)
- 7: Hohlraum (des Probeinlays)
- 8: Außenfläche (des Probeinlays)
- 9: Innenfläche (des Probeinlays)
- 10: Außenfläche
- 11: Innenfläche
- 12: Grundkörper
- 13: Halteelement
- 14: Abschnitte
- 15a-d: Steg
- 16: Pfannenöffnung
- 17: Zwischenräume
- 18: Polabschnitt
- 19: Aussparung (des Polabschnitts)
- 20: Proximales Ende
- 21: Distales Ende
- 22: Randbereich
- 23: Blattfeder
- 24: Erhebung
- 25: Aussparung (des Halteelements)
- 30 - 38: Block
- 41: Greifelement

## Patentansprüche

1. Selbstfixierende Probepfanne zum lösbaren Einsetzen in eine entsprechende Ausnehmung eines Hüftknochens, umfassend
- eine Außenfläche (10), die an die Ausnehmung des Hüftknochens angepasst ist,
- eine Innenfläche (11), die an ein Inlay, insbesondere Probeinlay, und/oder einen Kugelgelenkkopf zur Aufnahme desselben in der Hüftgelenkpfanne angepasst ist, und
- mindestens ein Halteelement (13) zum Fixieren der Hüftgelenkpfanne in der Ausnehmung,
**dadurch gekennzeichnet, dass**
das mindestens eine Halteelement (13) mindestens ein Federelement umfasst, das derart ausgebildet ist, dass das Federelement im eingesetzten Zustand eine nach Außen gerichtete Federkraft zumindest abschnittsweise auf die Ausnehmung des Hüftknochens zum lösbaren Fixieren der Hüftgelenkpfanne ausübt.

2. Selbstfixierende Probepfanne nach Anspruch 1,
**dadurch gekennzeichnet, dass**
das Federelement bzw. mindestens eines der Federelemente als Blattfeder (23) ausgebildet ist, die vorzugsweise zumindest bereichsweise die Außenfläche (10) der Hüftgelenkpfanne bildet.

3. Selbstfixierende Probepfanne nach einem der vorhergehenden Ansprüche,
**dadurch gekennzeichnet, dass**
ein proximales Ende (20) des Halteelements (13) in der Nähe des Durchstoßpunktes der Rotationssymmetrieachse der Hüftgelenkpfanne an dieser fixiert ist und ein in einem Randbereich (22) der Hüftgelenkpfanne befindliches, distales Ende (21) durch Druckbeaufschlagung in seiner Position veränderbar ist.

4. Selbstfixierende Probepfanne nach einem der vorhergehenden Ansprüche,
**dadurch gekennzeichnet, dass**
das/die Halteelemente (13) an einem dem Hüftknochen zugewandten Flächenabschnitt der Außenfläche Erhebungen (24), insbesondere zackenförmige Erhebungen aufweist/aufweisen.

5. Selbstfixierende Probepfanne nach einem der vorhergehenden Ansprüche,
**dadurch gekennzeichnet, dass**
die Erhebungen (24) an dem distalen Ende (21) des Halteelements (13) angeordnet sind.

6. Selbstfixierende Probepfanne nach einem der vorhergehenden Ansprüche,
**dadurch gekennzeichnet, dass**
ein Überstand der Hüftgelenkpfanne im eingesetzten Zustand derselben vorzugsweise mindestens 0,5cm, insbesondere mindestens 1,0cm über einen Rand der Ausnehmung des Hüftknochens hinausragt.

7. Selbstfixierende Probepfanne nach einem der vorhergehenden Ansprüche,
**dadurch gekennzeichnet, dass**
das Halteelement/die Halteelemente (13) im Bereich des Überstandes derartig ausgestaltet ist/sind, dass es/sie mit einem entsprechenden Werkzeug zum Lösen des Halteelements/der Halteelemente (13) in Eingriff bringbar ist/sind.

8. Selbstfixierende Probepfanne nach einem der vorhergehenden Ansprüche,
**dadurch gekennzeichnet, dass**
die Hüftgelenkpfanne durchgehende Aussparungen aufweist derart, dass eine gitterähnliche Struktur ausgebildet ist.

9. Anordnung, umfassend die selbstfixierende Probepfanne nach einem der vorhergehenden Ansprüche und ein Manipulierinstrument, wobei das Manipulierinstrument derart, insbesondere zangenartig ausgebildet ist, dass durch eine das Manipulierinstrument bedienende Person oder Vorrichtung eine Kraft auf die Halteelemente (13) aufbringbar ist, welche in das Innere der Hüftgelenkpfanne gerichtet ist.

10. Anordnung, umfassend die selbstfixierende Probepfanne nach einem der vorhergehenden Ansprüche und eine Positionsbestimmungshilfe und/oder Orientierungsbestimmungshilfe,
**dadurch gekennzeichnet, dass**
die Positionsbestimmungshilfe und/oder Orientierungsbestimmungshilfe zur Bestimmung und/oder Abspeicherung der Position und/oder Orientierung der Hüftgelenkpfanne an diese fixierbar ist/sind.

11. Anordnung, umfassend die selbstfixierende Probepfanne nach einem der vorhergehenden Ansprüche und ein Inlay, insbesondere Probeinlay, welches in die selbstfixierende Probepfanne einsetzbar ist.

12. Set, bestehend aus einer Vielzahl von selbstfixierenden Probepfannen nach einem der vorhergehenden Ansprüche
**dadurch gekennzeichnet, dass**
sich die einzelnen Hüftgelenkpfannen in ihrer Größe, insbesondere bezüglich ihres Kugelradius unterscheiden.

## Claims

1. A self-fixating trial socket for releasable insertion into a corresponding cavity in a hip bone, comprising:
an outer surface (10) matched to the cavity in the hip bone,
an inner surface (11) matched to an inlay, in particular a trial inlay, and/or to a ball joint head, for receiving the latter in the hip joint socket, and
at least one holding element (13) for fixing the hip joint socket in the cavity,
**characterized in that**
the at least one holding element (13) comprises at least one spring element which is configured such that in the inserted state the spring element exerts, at least in some places, an outwardly directed spring force to the cavity in the hip bone for releasable fixation of the hip joint socket.

2. The self-fixating trial socket according to claim 1,
**characterized in that**
the spring element or rather at least one of the spring elements is in the form of a leaf spring (23) which, preferably at least in some regions, forms the outer surface (10) of the hip joint socket.

3. The self-fixating trial socket according to one of the preceding claims,
**characterized in that**
a proximal end (20) of the holding element (13) is fixed to the hip joint socket in the vicinity of the piercing point of the axis of rotational symmetry thereof, and the position of the distal end (21) located in an edge region (22) of the hip joint socket is variable in response to pressure application.

4. The self-fixating trial socket according to one of the preceding claims,
**characterized in that**
the holding element(s) (13) has/have protrusions (24), especially sawtooth-like protrusions, on a surface portion of the outer surface, which surface portion faces the hip bone.

5. The self-fixating trial socket according to one of the preceding claims,
**characterized in that**
the protrusions (24) are arranged at the distal end (21) of the holding element (13).

6. The self-fixating trial socket according to one of the preceding claims,
**characterized in that**
in the inserted state of the hip joint socket, a protrusion thereof projects preferably by at least 0.5 cm, in particular by at least 1.0 cm, beyond the edge of the cavity in the hip bone.

7. The self-fixating trial socket according to one of the preceding claims,
**characterized in that**
the holding element(s) (13) in the region of the protrusion is/are configured such to be brought into engagement with a corresponding tool for releasing the holding element(s) (13).

8. The self-fixating trial socket according to one of the preceding claims,
**characterized in that**
the hip joint socket has through-apertures such that a grid-like structure is formed.

9. An arrangement comprising the self-fixating trial socket according to one of the preceding claims and a manipulation instrument, wherein the manipulation instrument being constructed such, especially forceps-like, to allow a person or device operating the manipulation instrument to apply to the holding elements (13) a force that is directed to the interior of the hip joint socket.

10. An arrangement comprising the self-fixating trial socket according to one of the preceding claims and a position-determining aid and/or an orientation-determining aid,
**characterized in that**
the position-determining aid and/or orientation-determining aid is/are fixable to the hip joint socket for determining and/or storing the position and/or orientation thereof.

11. An arrangement comprising the self-fixating test socket according to one of the preceding claims and an inlay, especially a trial inlay, which is insertable into the self-fixating test socket.

12. A set comprising a plurality of self-fixating trial sockets according to one of the preceding claims,
**characterized in that**
the individual hip joint sockets differ in size, especially in respect of their ball radius.

## Revendications

1. Cotyle test à auto-fixation destiné à être mis en place de façon détachable dans un évidement correspondant d'un os iliaque, comprenant
- une surface extérieure (10), qui est adapté à l'évidement de l'os iliaque,
- une surface intérieure (11) qui est adaptée à un inlay, en particulier un inlay test et/ou une tête d'articulation sphérique pour la réception de celle-ci dans le cotyle de la hanche, et
- au moins un élément de maintien (13) pour la fixation du cotyle de la hanche dans l'évidement,
**caractérisé en ce que**
ledit au moins un élément de maintien (13) inclut au moins un élément à ressort qui est réalisé de telle façon que l'élément à ressort exerce, dans la situation mise en place, une force élastique dirigée vers l'extérieur par portions sur l'évidement de l'os iliaque, pour la fixation détachable du cotyle de la hanche.

2. Cotyle test à auto-fixation selon la revendication 1,
**caractérisé en ce que** l'élément à ressort ou l'un au moins des éléments à ressort est réalisé sous forme de ressort à lame (23) qui forme de préférence au moins localement la surface extérieure (10) du cotyle de la hanche.

3. Cotyle test à auto-fixation selon l'une des revendications précédentes,
**caractérisé en ce qu'**une extrémité proximale (20) de l'élément de maintien (13) est fixée sur le cotyle de la hanche au voisinage du point de traversée de l'axe de symétrie de révolution du cotyle de la hanche, et une extrémité distale (21), qui se trouve dans une zone de bordure (22) du cotyle de la hanche, est modifiable quant à sa position par application d'une pression.

4. Cotyle test à auto-fixation selon l'une des revendications précédentes,
**caractérisé en ce que** l'élément/les éléments de maintien (13) comprend/comprennent des saillies (24), en particulier des saillies en forme de dents, sur un tronçon de la surface extérieure tournée vers l'os iliaque.

5. Cotyle test à auto-fixation selon l'une des revendications précédentes,
**caractérisé en ce que** les saillies (24) sont agencées à l'extrémité distale (21) de l'élément de maintien (13).

6. Cotyle test à auto-fixation selon l'une des revendications précédentes,
**caractérisé en ce que**, dans la situation mise en place du cotyle de la hanche, un surplomb de celui-ci dépasse de préférence d'au moins 0,5 cm, en particulier d'au moins 1,0 cm au-delà d'une bordure de l'évidement de l'os iliaque.

7. Cotyle test à auto-fixation selon l'une des revendications précédentes,
**caractérisé en ce que** l'élément/les éléments de maintien (13) est/sont conçu(s) dans la région du surplomb de telle manière qu'il(s) peut/peuvent être amené(s) en engagement avec un outil correspondant pour détacher l'élément/les éléments de maintien (13).

8. Cotyle test à auto-fixation selon l'une des revendications précédentes,
**caractérisé en ce que** le cotyle de la hanche comporte des échancrures traversantes de telle façon qu'il en résulte une structure semblable à une grille.

9. Agencement, incluant le cotyle-test à auto-fixation selon l'une des revendications précédentes, et un instrument de manipulation, dans lequel l'instrument de manipulation est réalisé, en particulier à la manière d'une pince, de telle façon une force peut être appliquée sur les éléments de maintien (13), par une personne ou un appareil qui utilise l'instrument de manipulation, force qui est orientée vers l'intérieur du cotyle de la hanche.

10. Agencement, incluant le cotyle test à auto-fixation selon l'une des revendications précédentes et un auxiliaire de détermination de position et/ou un auxiliaire de détermination d'orientation,
**caractérisé en ce que** l'auxiliaire de détermination de position et/ou l'auxiliaire de détermination d'orientation pour déterminer et/ou mémoriser la position et/ou l'orientation du cotyle de la hanche est/sont susceptible(s) d'être fixé(s) sur celui-ci.

11. Agencement, incluant le cotyle test à auto-fixation selon l'une des revendications précédentes, et un inlay, en particulier un inlay test, qui est susceptible d'être mis en place dans le cotyle test à auto-fixation.

12. Trousse, constituée d'une pluralité de cotyles test à auto-fixation selon l'une des revendications précédentes,
**caractérisée en ce que** les cotyles de la hanche individuels sont de tailles différentes, en particulier pour ce qui concerne leur rayon sphérique.
